# EUROPEAN PATENT SPECIFICATION

(11) **EP 2 844 390 B1**
(45) Date of publication and mention of the grant of the patent: **17.03.2021**
(21) Application number: 13719879.2
(22) Date of filing: 03.05.2013
(51) Int. Cl.: B01J 37/02, B01J 37/16, B01J 37/18, B01J 23/44, B01J 23/52, B01J 23/58, B01J 35/00, C07C 67/055

(54) **PRODUCTION OF SHELL CATALYSTS IN A COATING DEVICE**
HERSTELLUNG VON SCHALENKATALYSATOREN IN EINER BESCHICHTUNGSVORRICHTUNG
PRODUCTION DE CATALYSEURS EN COQUILLE DANS UN DISPOSITIF D'ENROBAGE

(30) Priority: 03.05.2012 DE 102012008715
(43) Date of publication of application: 11.03.2015
(73) Proprietor: Clariant International Ltd, 4132 Muttenz (CH)
(72) Inventor: MESTL, Gerhard, 80935 München (DE); SCHECK, Peter, 82205 Gilching (DE); HAGEMEYER, Alfred, Sunnyvale, California 94086 (US); FISCHER, Carolin, 83024 Rosenheim (DE)
(74) Representative: Kuba, Stefan
(86) International application number: PCT/EP2013/059262
(87) International publication number: WO 2013/164454

(56) References cited:
- US-A- 5 347 046
- US-A- 5 693 586
- US-A1- 2005 181 940
- US-A1- 2010 273 644

## Description

The present invention relates to a method for producing a shell catalyst which is suitable for the synthesis of alkenyl carboxylic acid esters, in particular for producing vinyl acetate monomers (VAM) from ethylene and allyl acetate monomers from propylene by means of oxy-acetylation.

Supported catalysts which contain palladium and gold have already been known for some time. VAM is usually produced in the presence of catalysts containing palladium and gold from a reaction mixture of ethylene, oxygen and acetic acid. Various production methods for such supported catalysts are already known. Thus, for example, precursor compounds which contain the corresponding metals are applied, dissolved preferably in an aqueous solution, to the surface of a support body. The support body containing the corresponding precursor compounds is then usually calcined under oxidizing conditions in a high-temperature oven, wherein the metal-containing precursor compounds are converted to the metal oxides. The support bodies which contain the corresponding metal oxides are then subjected to reduction to the elemental metals. In some known methods, however, precursor compounds are used in which an oxidation to the metal oxides is not necessary and the reduction step can be carried out directly after the drying. Vinyl acetate monomer is an important component for the production of polyvinyl acetate, vinyl acetate copolymers (such as ethylene vinyl acetates or ethylene vinyl alcohol copolymers) and polyvinyl alcohol. Because of the wide field of use of these polymers, for example as binders in the construction, paints, and varnishes sectors and as raw material for the adhesive, paper and textile industries, there is still a high demand for VAM and for constant improvement of the activity and selectivity of catalysts for their production.

Normally, in the synthesis of VAM, shell catalysts are used in which elemental palladium and gold are situated in an outer shell of the catalyst support body (hereafter called support body or shaped body). For their production, a mixed solution of a Pd-containing precursor compound and an Au-containing precursor compound is normally applied to support bodies in a coating device. Then, the support body is dried in a drying device. The metal components of the precursor compounds are then converted to the elemental metals in a reduction furnace. Then, the usually wet-chemical impregnation of the reduced support bodies with potassium acetate takes place. In these methods of the state of the art, the expenditure of time and the outlay on preparation are very high because of the introduction and removal of the support body into and from the devices used for the various production steps. High costs are also associated with the high expenditure of time. In addition, the decanting between the method steps carried out in the various devices subjects the support bodies to a strong mechanical stress which causes high abrasion. However, the high abrasion can, on the one hand, lead to blockage of the pores of the support body due to the formation of dust and, on the other hand, it leads to abrasion of the catalytically active shell of the cost-intensive noble metals situated therein. In addition, due to the capital intensity of VAM and allyl acetate production plants and increasingly high raw material costs, in particular for ethylene and propylene, there is a constant requirement to optimize the economic efficiency of the method for producing VAM by means of improved catalysts.

Furthermore, all the conventional methods for producing vinyl acetate and allyl acetate catalysts have proved to be capable of improvement in respect of the noble metal yield. The ratio between the proportion of noble metal, thus Pd and Au, which ultimately remains on the catalyst during the production process of the latter and the proportion of noble metal used is considered to be the noble metal yield. The catalyst intermediate products which are already covered with noble metals usually undergo further production steps, such as for example chemical fixing, washing, reducing and finally the application of the alkali acetate. Each of these subsequent production steps as well as the handling, necessary for this, in different containers and units inevitably leads to noble metal losses.

The object of the present invention was therefore to provide a method for producing a shell catalyst which makes possible a more cost-effective production of the catalysts that is more effective in terms of time and preparation. In addition, it was also an object to reduce the noble metal loss during the production of VAM shell catalysts (hereafter "VAM" means, not only vinyl acetate monomer, but also allyl acetate monomer). Moreover, it was also an object to provide a shell catalyst which outperforms previous catalysts in respect of the activity and selectivity in the synthesis of alkenyl carboxylic acid esters.

These objects were achieved by a method according to the invention for producing a shell catalyst which is specified in claim 1. By carrying out all the steps a to e in one device, the production time for the catalyst is greatly reduced and the noble metal loss can also be greatly minimized, as the support bodies are not subject to friction or associated metal abrasion, as is the case when they have had to be decanted into separate drying and reduction devices.

By the term "shell catalyst" is meant a catalyst which comprises a support body and a shell with catalytically active material, wherein the shell can be formed in two different ways: Firstly, a catalytically active material can be present in the outer area of the support body, with the result that the material of the support body serves as matrix for the catalytically active material and the area of the support body which is impregnated with the catalytically active material forms a shell around the unimpregnated core of the support body. Secondly, an additional layer in which a catalytically active material is present can be applied to the surface of the support body. This layer thus forms an additional material layer which is constructed as a shell around the support body. In the latter variant, the support body material is not a constituent of the shell, but the shell is formed by the catalytically active material itself or a matrix material which comprises a catalytically active material. In the present invention, the first-named variant of a shell catalyst is preferred.

In the shell catalyst produced by the method according to the invention, the metals are present either in monoatomic form or in the form of aggregates. However, they are preferably present in the form of aggregates. The monoatomic atoms or multiatomic aggregates are dispersed predominantly uniformly inside the shell of the shell catalyst. By a multiatomic aggregate is meant the clustering of several metal atoms to form a composite which lies between monoatomic form and metallic type (alloy). The term also includes so-called metal clusters.

The shell thickness of the outer shell of the support body is preferably 1 to 50%, more preferably 2 to 40%, even more preferably 3 to 30% and most preferably 4 to 20% of half of the total thickness of the support body. The named percentage therefore relates to half of the total thickness as, depending on the shape of the support body during production, e.g. by spray impregnation with a solution containing precursor compound, the precursor compound either penetrates the support body material from two outer surfaces (sphere) or, if the support body material has a more complex shape, such as e.g. that of a hollow cylinder, there are an outer surface and an inner surface which the precursor compound penetrates. In the case of support body materials deviating from sphere geometry the total thickness of the support is measured along the longest support body axis. The outer shell boundary is equalized with the outer boundary of the metal-containing support body. By inner shell boundary is meant the boundary, located inside the support body, of the metal-containing shell which is at such a distance from the outer shell boundary that 95 wt.-% of all of the metal contained in the support body is located in the outer shell. However, the shell thickness is preferably not more than 50%, more preferably not more than 40%, even more preferably not more than 30% and most preferably not more than 20%, in each case relative to half of the total thickness of the support body.

The metal-impregnated support body preferably contains no more than 5% of the total metal in its inner area, thus inside the area that is delimited to the outside by the inner shell boundary of the metal shell.

With regard to the shell thickness of the catalyst, the maximum concentration of metal preferably lies in the area of the outer shell, particularly preferably at the outer edge of the outer shell, i.e. close to the geometric catalyst surface. The metal concentration preferably decreases towards the inner shell boundary.

The support body preferably consists of an inert material. It can be porous or non-porous. However, the support body is preferably porous. The support body preferably consists of particles with a regular or irregular shape, such as for example spheres, tablets, cylinders, solid cylinders or hollow cylinders, rings, stars or other shapes, and its dimensions, such as e.g. diameter, length or width, are in a range of from 1 to 10 mm, preferably 3 to 9 mm. Spherical, i.e. e.g. sphere-shaped, particles with a diameter of from 3 to 8 mm are preferred according to the invention. According to the invention, the support body comprises an Si-Al mixed oxide. The support body, an Si-Al mixed oxide, can in addition also be doped with Zr and preferably contains this in a proportion of from 5 to 30 wt.-%, relative to the total weight of the support body.

The BET surface area of the support body material without the coating with the precursor compounds is 1 to 1,000 m²/g, preferably 10 to 600 m²/g, particularly preferably 20 to 400 m²/g and quite particularly preferably between 80 and 170 m²/g. The BET surface area is determined using the 1-point method by adsorption of nitrogen in accordance with DIN 66132.

In addition, it can be preferred that the integral pore volume of the support body material (determined in accordance with DIN 66133 (Hg porosimetry)) without the coating with the precursor compound is greater than 0.1 ml/g, preferably greater than 0.18 ml/g.

The support body is usually produced by subjecting a plurality of support bodies to a "batch" process, in the individual method steps of which the shaped bodies are subject to relatively high mechanical stresses for example by using stirring and mixing tools. In addition, the shell catalyst produced by the method according to the invention can be subjected to a strong mechanical load stress during the filling of a reactor, which can result in an undesired formation of dust as well as damage to the support body, in particular to its catalytically active shell located in an outer area.

In particular, to keep the abrasion of the catalyst produced by the method according to the invention within reasonable limits, the shell catalyst has a hardness greater than/equal to 20 N, preferably greater than/equal to 25 N, further preferably greater than/equal to 35 N and most preferably greater than/equal to 40 N. The hardness is ascertained by means of an 8M tablet-hardness testing machine from Dr. Schleuniger Pharmatron AG, determining the average for 99 shell catalysts, after drying of the catalyst at 130°C for 2 hours, wherein the apparatus settings are as follows:
Distance from the shaped body: 5.00 mm
Time delay: 0.80 s
Feed type: 6 B
Speed: 0.60 mm/s

The hardness of the shell catalyst produced by the method according to the invention can be influenced for example by means of variations in certain parameters of the method for producing the support body, for example by the calcining time and/or the calcining temperature of the support body. The just-mentioned calcining is not a calcining of the support body impregnated with the metal-containing precursor compounds, but merely a calcining step for producing the support body before the precursor compounds are applied.

It is also preferred that 80% of the integral pore volume of the support body is formed by mesopores and macropores, preferably at least 85% and most preferably at least 90%. This counteracts a reduced activity, effected by diffusion limitation, of the catalyst produced by the method according to the invention, in particular in the case of metal-containing shells with relatively large thicknesses. By micropores, mesopores and macropores are meant in this case pores which have a diameter of less than 2 nm, a diameter of from 2 to 5 nm and a diameter of more than 50 nm respectively.

The activity of the shell catalysts produced by the method according to the invention normally depends on the quantity of the metal loading in the shell: As a rule, the more metal there is in the shell, the higher the activity. The thickness of the shell here has a smaller influence on the activity, but is a decisive variable with respect to the selectivity of the catalysts. With equal metal loading of the catalyst support, the smaller the thickness of the outer shell of the catalyst is, the higher the selectivity of the shell catalysts produced by the method according to the invention is in general. It is thus decisive to set an optimum ratio of metal loading to shell thickness in order to guarantee the highest possible selectivity with the highest possible activity. It is therefore preferred that the shell of the shell catalyst produced according to the invention has a thickness in the range of from 20 µm to 1,000 pm, more preferably from 30 µm to 800 pm, even more preferably from 50 µm to 500 µm and most preferably from 100 µm to 300 µm.

The thickness of the shell can be measured visually by means of a microscope. The area in which the metal is deposited appears black, while the areas free of noble metals appear white. As a rule, in the case of shell catalysts produced according to the invention the boundary between areas containing noble metals and areas free of them is very sharp and can be clearly recognized visually. If the above-named boundary is not sharply defined and accordingly not clearly recognizable visually, the thickness of the shell corresponds - as already mentioned - to the thickness of a shell, measured starting from the outer surface of the catalyst support, which contains 95% of the noble metal deposited on the support. In order to ensure a largely uniform activity of the catalyst produced by the method according to the invention over the thickness of the noble metal-containing shell, the noble-metal concentration should vary only relatively little over the shell thickness. It is therefore preferred if, over an area of 90% of the shell thickness, wherein the area is at a distance of 5% of the shell thickness from each of the outer and inner shell limits, the profile of the noble-metal concentration of the catalyst varies from the average noble-metal concentration of this area by a maximum of +/- 20%, preferably by a maximum of +/- 15% and by preference by a maximum of +/- 10%. Such profiles can be achieved for example by means of physical deposition methods, such as spray coating a solution containing the precursor compound onto support bodies circulated in a gas. The support bodies here are preferably located in a so-called fluidized bed or in a fluid bed, but all devices in which the support bodies can be swirled in a gas glide layer are also conceivable. The named shell profiles can particularly preferably be obtained by means of the spraying-on described further below in a fluidized bed, a fluid bed or an Innojet AirCoater. In the case of the shell catalysts produced according to the invention, the named distribution of the metal loading preferably describes a rectangular function, i.e. the concentration does not decrease or only decreases imperceptibly during the course into the inside of the support body and ends with a relatively "sharp" boundary (see above-named distribution parameters). In addition to the rectangular function, the metal loading inside the shell can however also describe a triangular or trapezium function in the case of which the metal concentration gradually decreases from the outside to the inside in the shell. However, a metal distribution according to the rectangular function is particularly preferred.

In step (a) of the method according to the invention, a support body is first introduced into a coating device. The method according to the invention is characterized in that all the steps (b) to (d) are carried out in the named coating device, without the support bodies being removed from the coating device during steps (a) to (e). This has the advantage that all the coating steps can be carried out in one device, which makes the method time- and cost-effective, and reduces the metal abrasion.

The coating device is preferably a device in which the support bodies can be swirled in a glide layer of process gas and into which the solutions of the components named in step (b) of the method according to the invention can be sprayed. In addition, the device should preferably have a feed of process or reduction gas, and a heating device. In a preferred embodiment, the coating device is constituted by conventional coating drums, fluidized bed devices or fluid bed devices.

Suitable conventional coating drums, fluidized bed devices or fluid bed devices for carrying out the application of the precursor compound in the method according to the invention are known in the state of the art and are marketed for example by companies such as Heinrich Brucks GmbH (Alfeld, Germany), ERWEKA GmbH (Heusenstamm, Germany), Stechel (Germany), DRIAM Anlagenbau GmbH (Eriskirch, Germany), Glatt GmbH (Binzen, Germany), G. S. Divisione Verniciatura (Osteria, Italy), HOFER-Pharma Maschinen GmbH (Weil am Rhein, Germany), L.B. Bohle Maschinen und Verfahren GmbH (Enningerloh, Germany), Lödige Maschinenbau GmbH (Paderborn, Germany), Manesty (Merseyside, Great Britain), Vector Corporation (Marion (IA) USA), Aeromatic-Fielder AG (Bubendorf, Switzerland), GEA Process Engineering (Hampshire, Great Britain), Fluid Air Inc. (Aurora, Illinois, USA), Heinen Systems GmbH (Varel, Germany), Hüttlin GmbH (Steinen, Germany), Umang Pharmatech Pvt. Ltd. (Maharashtra, India) and Innojet Technologies (Lörrach, Germany). Particularly preferred fluid bed equipment is sold with the name Innojet® AirCoater or Innojet® Ventilus by Innojet Technologies. Here the IAC-5 coater, the IAC-150 coater or the IAC-025 coater, all from the company Innojet, is particularly preferably used.

After the introduction of the support body into the coating device, dust is preferably first removed from it by swirling a bed of the support bodies through process gas, i.e. the support bodies are preferably held in an air-glide layer produced by the process gas. This step is preferably carried out at a temperature in the range of from 20 to 50°C. The duration of the dust removal is preferably 1 minute to 15 minutes.

It is preferred in the method according to the invention that step (b) is carried out by simultaneous application of the Pd precursor compound and the Au precursor compound.

The Pd precursor compounds and Au precursor compounds used in the method according to the invention are preferably water-soluble compounds.

The Pd precursor compound used in the method according to the invention is preferably selected from: nitrate compounds, nitrite compounds, acetate compounds, tetraamine compounds, diamine compounds, hydrogen carbonate compounds and hydroxidic metallate compounds.

Examples of preferred Pd precursor compounds are water-soluble Pd salts. The Pd precursor compound is particularly preferably selected from the group consisting of Pd(NH₃)₄(HCO₃)₂, Pd(NH₃)₄(HPO₄), ammonium Pd oxalate, Pd oxalate, K₂Pd(oxalate)₂, Pd(II) trifluoroacetate, Pd(NH₃)₄(OH)₂, Pd(NO₃)₂, H₂Pd(OAc)₂(OH)₂, Pd(NH₃)₂(NO₂)₂, Pd(NH₃)₄(NO₃)₂, H₂Pd(NO₂)₄, Na₂Pd(NO₂)₄, Pd(OAc)₂ as well as freshly precipitated Pd(OH)₂.

If freshly precipitated Pd(OH)₂ is used, it is preferably produced as follows: A 0.1 to 40 wt.-% aqueous solution is preferably produced from tetrachloropalladate. A base, preferably an aqueous solution of potassium hydroxide, is then added to this solution until a brown solid, namely Pd(OH)₂ precipitates. To produce a solution for application to the catalyst support, the freshly precipitated Pd(OH)₂ is isolated, washed and dissolved in an aqueous alkaline solution. Dissolution preferably takes place at a temperature within the range of from 4 to 40°C, particularly preferably 15 to 25°C. A lower temperature is not possible because of the freezing point of water, a higher temperature brings with it the disadvantage that after a certain time Pd(OH)₂ precipitates again in the aqueous solution and does not dissolve.

A solution of the compound Pd(NH₃)₄(OH)₂ is preferably produced as follows: A precursor compound such as for example Na₂PdCl₄ is - as previously described - precipitated with potassium hydroxide solution to palladium hydroxide, preferably at pH 11 and room temperature, and the precipitate, after filtration and washing, is dissolved in aqueous ammonia (maximum 8.5%) to form Pd(NH₃)₄(OH)₂ (approx. 30 minutes at room temperature, approx. 2 hours at 60°C).

Furthermore the Pd-nitrite precursor compounds can also be used in the method according to the invention. Preferred Pd-nitrite precursor compounds are for example those which are obtained by dissolving Pd(OAc)₂ in an NaNO₂ or KNO₂ solution.

However, the above-named hydroxo complexes or hydroxo compounds are particularly preferably used as Pd precursor compounds. The compound Pd(NH₃)₄(OH)₂ is quite particularly preferably used as Pd precursor compound.

The Au precursor compounds used in the method according to the invention are preferably selected from: acetate compounds, nitrite or nitrate compounds and oxidic or hydroxidic metallate compounds.

Examples of preferred Au precursor compounds are water-soluble Au salts. The Au precursor compound is preferably selected from the group consisting of KAuO₂, NaAuO₂, LiAuO₂, RbAuO₂, CsAuO₂, Ba(AuO₂)₂, NaAu(OAc)₃(OH), KAu(NO₂)₄, KAu(OAc)₃(OH), LiAu(OAc)₃(OH), RbAu(OAc)₃(OH), CsAu(OAc)₃(OH), HAu(NO₃)₄ and Au(OAc)₃. It may be advisable to add the Au(OAc)₃ or one of the named aurates in each case freshly by precipitation as oxide or hydroxide from an auric acid solution, washing and isolating the precipitate as well as taking up same in acetic acid or alkali hydroxide respectively. One of the named alkali aurates is particularly preferably used as Au-containing precursor compound, which is used in dissolved form for application to the support. The production of a potassium aurate solution is known in the literature and can be carried out in accordance with the production methods disclosed in the documents WO99/62632 and US 6,015,769. The other alkali aurates can also be produced analogously to this. It is particularly preferred that CsAuO₂ or its hydroxide dissolved in water (CsAu(OH)₄) is used as Au precursor compound in the method according to the invention. In particular, the use of CsAuO₂ means that the Au precursor compound does not penetrate further into the support body than the Pd precursor compound, which makes possible a uniform distribution of the two components in the shell.

The named precursor compounds are mentioned herein only by way of example and any further precursor compounds can be used which are suitable for the production of a VAM shell catalyst. It is particularly preferred that the precursor compounds are substantially chloride-free. By substantially chloride-free is meant that the empirical formula of the compound comprises no chloride, but it is not ruled out that the compound contains unavoidable chloride impurities for example due to production conditions.

It is particularly preferred that the Pd precursor compound in step (b) is the compound Pd(NH₃)₄(OH)₂, and the Au precursor compound is CsAuO₂.

In step (b) of the method according to the invention the Pd precursor compound and the Au precursor compound are present dissolved in a solvent. The Pd precursor compound and the Au precursor compound can be present dissolved in a mixed solution, but they can also each be present in a separate solution. Pure solvents and solvent mixtures in which the selected metal compound(s) is/are soluble and which, after application to the catalyst support, can be easily removed again from same by means of drying are suitable as solvents for the transition metal precursor compounds. Preferred solvents are unsubstituted carboxylic acids, in particular acetic acid, ketones, such as acetone, and in particular deionized water.

In step (b) the precursor compounds can be applied either from a mixed solution containing the Au precursor compound and the Pd precursor compound or from two solutions each containing one of the two precursor compounds. The precursor compounds are particularly preferably applied to the support body in step (b) simultaneously from two different solutions. If the Au precursor compound and the Pd precursor compound are applied from a mixed solution, the mixed solution is preferably conveyed from a receiver container via a pump to a spray nozzle via which the precursor compounds are sprayed onto the support bodies. If the Au precursor compound and the Pd precursor compound are applied from two separate solutions, it is preferred that these are stored in two separate receiver containers. They can then be conveyed from these by means of two pumps to two spray nozzles, with the result that the two solutions are sprayed in separately. Alternatively, the separate solutions can also be conveyed by means of two pumps to one spray nozzle, with the result that both solutions are sprayed in via one nozzle.

The application of the precursor compounds in step (b) of the method according to the invention is carried out by spraying the support body with a solution containing the precursor compound. It is particularly preferred that the movement of the support bodies during the spray coating is carried out with the help of a support gas (or process gas), for example in a fluid bed, a fluidized bed or in an Innojet AirCoater, wherein hot air is preferably blown in, with the result that the solvent is quickly evaporated. In this way, the precursor compounds are present in the named defined shell of the support body. The spraying rate is preferably chosen during the spraying such that a balance is achieved between the evaporation rate of the solvent and the feed rate of the precursor compounds on the support body. This makes it possible to set the desired shell thickness and palladium/gold distribution in the shell. Depending on the spraying rate, the shell thickness can thus be infinitely variably set and optimized, for example up to a thickness of 2 mm. But very thin shells with a thickness in the range of from 50 to 300 µm are thus also possible.

The solution(s) containing the metal precursor compounds is/are preferably sprayed through a spray nozzle into the apparatus, in which the spray gas fed in, preferably a gas with a non-reductive action, such as air or an inert gas, is fed in at a pressure in the range of from 1 to 1.8 bar, more preferably 1.0 to 1.6 bar and most preferably 1.1 to 1.3 bar. Process air is preferably used as process gas for circulating the support bodies. The spraying rate and the pressure of the spray gas is preferably chosen for the nozzle used such that when it meets the circulating support bodies the droplet size of the resultant aerosol is between 1 and 100 pm, preferably between 10 and 40 µm. For example an IRN10 PEEK-type Rotojet spray nozzle from Innojet can be used here.

It is particularly preferred that the spraying rate in step (b) during the application of the metal precursor compounds is constant and, depending on the precursor compound, is in the range of a mass flow (the solution containing the precursor compound) of from 5 g/min per 100 g to 25 g/min per 100 g of support body to be coated, more preferably in the range of from 10 to 20 g/min per 100 g and most preferably in the range of from 13 to 17 g/min per 100 g. In other words the ratio of the weight of the sprayed-on solution to the weight of the packed bed of the support body lies in the range of from 0.05 to 0.25, more preferably 0.1 to 0.2 and most preferably 0.13 to 0.17. A mass flow or ratio above the range indicated leads to catalysts with lower selectivity, a mass flow or ratio below the range indicated has no marked negative effects on the catalyst performance, but the catalyst production is more time-consuming and the production is thus less efficient.

If a fluid bed device is used as coating device in the method according to the invention, it is preferred if the support bodies circulate elliptically or toroidally in the fluid bed. To give an idea of how the support bodies move in such fluid beds, it may be stated that in the case of "elliptical circulation" the support bodies move in the fluid bed in a vertical plane on an elliptical path, the size of the main and secondary axes changing. In the case of "toroidal" circulation the support bodies move in the fluid bed in a vertical plane on an elliptical path, the size of the main and secondary axes changing, and in a horizontal plane on a circular path, the size of the radius changing. On average, the support bodies move in a vertical plane on an elliptical path in the case of an "elliptical circulation", on a toroidal path in the case of a "toroidal circulation", i.e. a support body travels helically over the surface of the torus with a vertically elliptical section.

It is particularly preferred during the application of the compounds in step (b) of the method according to the invention that there is a so-called controlled air-glide layer in the unit. For one thing, the support bodies are thoroughly mixed by the controlled air-glide layer, wherein they simultaneously rotate about their own axis, and are dried evenly by the process air. For another, due to the consistent orbital movement, effected by the controlled air-glide layer, of the support bodies the support bodies pass through the spray procedure (application of the precursor compounds) at a virtually constant frequency. A largely uniform shell thickness, or penetration depth of the metals into the support body, of a treated phase of support bodies is thereby achieved. A further result is that the noble-metal concentration varies only relatively slightly over a relatively large area of the shell thickness, i.e. the noble-metal concentration describes an approximately rectangular function over a large area of the shell thickness, whereby a largely uniform activity of the resulting catalyst is guaranteed over the thickness of the noble metal shell.

Furthermore, the support body used in the method according to the invention is preferably heated during the spray coating of the metal precursor compounds in step (b), for example by means of heated process gas. The process gas here preferably has a temperature of from 10 to 110°C, more preferably 40 to 100°C and most preferably 50 to 90°C. The named upper limits should be adhered to in order to guarantee that the named outer shell has a small layer thickness with a high concentration of noble metal.

Air is preferably used in each case as process gas in this application. However, inert gases such as for example nitrogen, CO₂, helium, neon, argon or mixtures thereof can also be used.

If the Pd precursor compound and Au precursor compound in step (b) are applied from one solution, the solution preferably contains a proportion of Pd precursor compound such that Pd lies in the range of from 0.1 to 5 wt.-%, more preferably in the range of from 0.3 to 2 wt.-% and most preferably in the range of from 0.5 to 1 wt.-%, and a proportion of Au-containing precursor compound such that the proportion of Au lies in the range of from 0.05 to 10 wt.-%, more preferably in the range of from 0.1 to 5 wt.-% and most preferably in the range of from 0.1 to 1 wt.-%, relative to the atomic weight proportion of the metals in solution.

If the Pd precursor compound and the Au precursor compound are applied separately from different solutions, the Pd-containing solution preferably contains Pd in the range of from 0.1 to 10 wt.-%, more preferably in the range of from 0.2 to 5 wt.-% and most preferably in the range of from 0.5 to 1 wt.-%, and the Au-containing solution preferably contains Au in the range of from 0.1 to 15 wt.-%, more preferably in the range of from 0.2 to 5 wt.-% and most preferably in the range of from 0.3 to 1 wt.-%, in each case relative to the atomic weight proportion of the metals in solution.

After the step of applying the metal precursor compounds to the support body in step (b), a drying step (c) takes place before the reducing step (d). The drying step is preferably carried out below the decomposition temperature of the precursor compounds, in particular at a temperature in the range of from 70 to 120°C, more preferably 80 to 110°C and most preferably 90 to 100°C. The duration of the drying of the support body loaded with the metal precursor compounds preferably lies in the range of from 10 to 100 minutes, more preferably 30 to 60 minutes. By a decomposition temperature is meant the temperature at which the precursor compounds start to decompose. The drying preferably takes place using process gas. If the drying is carried out in the fluid bed device or the fluidized bed device, it is preferred that the support bodies are present static in the device, i.e. are not swirled by process gas. The step of drying in the coating device - and not in a separate drying oven - has the advantage that the support bodies loaded with the metal precursor compounds are not subject to mechanical strain due to the decanting into a further apparatus. The noble metal abrasion is therefore less, and likewise time is saved. In addition, the applicants of the present application have found that there is no disadvantage if the support bodies are not swirled during the drying, but the drying takes place statically by passing the process gas through the coating apparatus. The advantage of the static drying is the lower mechanical strain and the associated lower noble metal loss.

In addition to the step of simultaneously applying an Au precursor compound and a Pd precursor compound in step (b) of the method according to the invention, a pregilding can take place beforehand and/or an aftergilding of the support body afterwards. The step of pregilding or the step of aftergilding is preferably carried out in the same way as in the step of simultaneously applying the precursor compounds in step (b). Here, the same concentrations and the same precursor compounds as in the production of a solution produced separately in step (b) containing the Au precursor compound are preferably used. Here too, application by spray coating, as specified further above for the simultaneous application of the metal precursor compounds in step (b), is preferred. It is particularly preferred that the pregilding or aftergilding takes place by spray coating onto support bodies fluidized in a fluid bed or in a fluidized bed, as disclosed further above preferably also for step (b).

If a pregilding is carried out, an optional drying step, as specified further above, can be carried out after this. A step of intermediate calcining can also be carried out after the pregilding, before the step of simultaneously applying the metal precursor compounds in step (b) is carried out. Likewise, in the case of the aftergilding after the application of the metal precursor compounds in step (b), an identical drying or intermediate calcining step - as specified for the pregilding - can be carried out.

However, if a pre- and/or aftergilding is/are carried out, it is particularly preferred that this/these is/are carried out immediately before or immediately after the simultaneous application of the precursor compounds in step (b). In this case, it is particularly preferred that the Pd precursor compound and the Au precursor compound are applied in separate solutions during the simultaneous application in step (b). Thus, for example, for a desired pregilding during the spray coating the Au precursor compound solution can be sprayed in to start with and the spraying-in of the Pd precursor compound solution can be initiated after a certain amount of time (while Au precursor compound solution continues to be sprayed on). In a similar way, the aftergilding can be carried out by spray coating such that during the application of the metal precursor compounds in step (b) two separate solutions are sprayed in and the spraying-in of the Pd precursor compound solution ends before the spraying-in of the Au precursor compound solution is stopped.

The pregilding has the advantage that during the simultaneous application of the two precursor compound solutions in step (b) these precursor compounds already attach to the applied Au precursor compound in the support body. In particular, the Au precursor compound solution is thereby prevented from penetrating further into the support body than the Pd precursor compound, with the result that both metals have an almost identical penetration depth in the finished catalyst. In the specified pregilding or aftergilding by spray coating, preferably the same specifications for the concentration of the Au precursor compound in the solution, the same spraying rate, the same spraying pressure, the same spraying air and the same process gas apply as in the simultaneous application of the Pd and Au precursor compounds of the method according to the invention.

If an aftergilding is carried out, the step of drying the support body is carried out, preferably not immediately after the simultaneous application of the Pd and Au precursor compounds, but preferably only after the step of applying the additional Au precursor compound in the step of aftergilding.

If the pregilding is carried out by spray coating in the preferably specified way before the step of simultaneously applying the Pd and Au precursor compounds, it is preferred that the spraying rate, the spraying pressure and the concentration of the solution do not change, or only change within the specified ranges, during the spraying-in of the Au precursor compound when the spraying-in of the Pd precursor compound solution begins. The ratio of the time interval of the simultaneous spraying-in of the two metal precursor compounds in step (b) to the time interval of the spraying-in of the Au precursor compound solution in the step of pre-impregnation preferably lies in the range of from 8 to 1, more preferably in the range of from 6 to 2 and most preferably in the range of from 5 to 3.

If an aftergilding is carried out by spray coating, the ratio of the time interval of the simultaneous spraying-in of the two precursor compound solutions in step (b) to the time interval of the spraying-in of the Au precursor compound during the step of post-impregnation lies in the range of from 8 to 1, more preferably in the range of from 6 to 2 and most preferably in the range of from 5 to 3. Here too, the spraying rate, the spraying pressure and the concentration of the Au precursor compound solution preferably do not change, or only change within the specified ranges, after the end of the spraying-in of the Pd precursor compound solution.

The spraying-on of the solutions containing the precursor compounds is effected in all the method steps of the method according to the invention preferably by atomizing the solution with the help of a spraying nozzle. Here, an annular gap nozzle is preferably used which sprays a spray cloud the plane of symmetry of which preferably runs parallel to the plane of the device base. Due to the 360° circumference of the spray cloud, the shaped bodies falling centrally can be sprayed particularly evenly with the solution. The annular gap nozzle, i.e. its orifice, is preferably completely embedded in the apparatus carrying out the circulating movement of the support bodies.

According to a further preferred embodiment of the method according to the invention, it is provided that the annular gap nozzle is centrally arranged in the base of the apparatus carrying out the circulating movement of the support bodies and the orifice of the annular gap nozzle is completely embedded in the apparatus. It is thereby guaranteed that the free path of the drops of the spray cloud until they meet a shaped body is relatively short and, accordingly, relatively little time remains for the drops to coalesce into larger drops, which could work against the formation of a largely uniform shell thickness.

The reduction of the metal components of the metal precursor compounds to the elemental metals in step (d) is likewise carried out in the coating device. This likewise has the advantage that the coated support bodies are not subject to mechanical strain and thus a loss of metal.

It is particularly preferred that the support bodies are not moved during the step of reducing the metal components, i.e. are present static in the coating device. In this way, the metal loss can be greatly reduced.

The step of reduction is preferably carried out by a temperature treatment in a non-oxidizing atmosphere for the reduction of the metal components of the precursor compounds to the elemental metals. The temperature treatment in a non-oxidizing atmosphere is preferably carried out in a temperature range of from 40 to 400°C, more preferably 50 to 200°C and most preferably 60 to 150°C.

By a non-oxidizing atmosphere is meant in the present invention an atmosphere which contains no, or almost no, oxygen or other gases with an oxidizing action. The non-oxidizing atmosphere can be an atmosphere of inert gas or a reducing atmosphere. A reducing atmosphere can be formed by a gas with a reductive action or a mixture of gas with a reductive action and inert gas.

In a variant of the method according to the invention, the reduction is carried out in an inert gas atmosphere. In this case, the counterions of the metal in the metal-containing precursor compound have a reductive action. A person skilled in the art is sufficiently aware which counterions can have a reductive action in this case.

In a further variant of the method according to the invention, the temperature treatment can be carried out directly in a reducing atmosphere. In this case, the precursor compounds are decomposed at the same temperature of the temperature treatment and the metal component is reduced to the elemental metals.

In yet another variant of the method according to the invention, the temperature treatment is preferably carried out such that there is a change from an inert gas atmosphere to a reducing atmosphere during the temperature treatment. The precursor compounds are first decomposed at their decomposition temperature in an inert gas atmosphere and then the metal components are reduced to the elemental metals by the change to a reducing atmosphere. The temperature during the decomposition under inert gas preferably lies in the range of from 200 to 400°C. The temperature during the subsequent reduction then preferably lies in the above-specified range.

It is particularly preferred that the change from an inert gas atmosphere to a reducing atmosphere is carried out such that the temperature during the change does not fall below the temperature desired for the reduction. N₂, He, Ne, Ar or mixtures thereof for example are used as inert gas. N₂ is particularly preferably used.

The component with a reductive action in the reducing atmosphere is normally to be selected depending on the nature of the metal component to be reduced, but preferably selected from the group of gases or vaporizable liquids consisting of ethylene, hydrogen, CO, NH₃, formaldehyde, methanol, formic acid and hydrocarbons, or is a mixture of two or more of the above-named gases/liquids. The reducing atmosphere particularly preferably comprises hydrogen as reducing component. It is preferred in particular if the reducing atmosphere is formed from forming gas, a mixture of N₂ and H₂. The hydrogen content is in the range of from 1 vol.-% to 15 vol.-%. The reduction in the method according to the invention is made for example with hydrogen (4 to 5 vol.-%) in nitrogen as process gas at a temperature in the range of from 60 to 150°C over a period of for example from 0.25 to 10 hours, preferably 2 to 6 hours.

The change named in the second method alternative from inert gas to a reducing atmosphere during the step of reduction preferably takes place by feeding one of the named reducing components into an inert gas atmosphere. Hydrogen gas is preferably fed in. The feeding of a gas with a reductive action to the inert gas has the advantage that the temperature does not fall substantially, or not down to or below the lower limit of 60°C desired for the reduction, with the result that there is no need for another cost- and energy-intensive heating necessitated by a corresponding total atmosphere exchange.

The catalyst support obtained after the reduction preferably contains a proportion of Pd in the range of from 0.5 to 2.5 wt.-%, more preferably in the range of from 0.7 to 2 wt.-%, even more preferably in the range of from 0.9 to 1.5 wt.-%, relative to the total weight of the catalyst support body.

The catalyst support body obtained after the reduction preferably contains a proportion of Au in the range of from 0.1 to 1.0 wt.-%, more preferably in the range of from 0.2 to 0.8 wt.-% and most preferably in the range of from 0.3 to 0.6 wt.-%, relative to the total weight of the catalyst support body.

It is particularly preferred that all the optional steps, such as the pre- or aftergilding and the drying, are also carried out in the coating device before the support body is removed from the coating device in step (e).

The support body remains in the coating device between steps (a) and (e) of the method according to the invention, i.e. is not removed from the device for any intermediate treatment and re-introduced. Carrying out all the method steps in one device leads to a substantial saving of time - and thus of costs - during the production of VAM catalysts compared with conventional production methods. In addition, the metal abrasion is kept within reasonable limits. Compared with conventional production methods in which the metal yield is at most 94%, the metal yield in the method according to the invention can be increased to 97%. This was not to be expected in particular due to the mechanical strain on the support bodies caused during the movement in the fluid bed.

To produce a catalyst by the method according to the invention, an alkali acetate is applied to the support body before step (b). According to the invention, the application of the alkali acetate is carried out before the application of the metal precursor compounds, in order that, after the application of the metal precursor compounds, the mechanical strain on the support bodies and thus the noble metal loss are kept as low as possible. The step of applying the alkali acetate is carried out before step (b) of the method.

After the application of the alkali acetate, a drying step preferably takes place which is preferably carried out by the support bodies not being swirled in a process gas, but being located static in a device. The drying conditions are named further below.

Contrary to the views held until now with respect to the production of VAM catalysts, the applicants of the present application have surprisingly discovered that the application of an alkali acetate to a support body before the application of the metal precursor compounds and before the reduction of the metal components of the precursor compounds leads to a VAM shell catalyst which has a much higher activity and selectivity than shell catalysts in which the alkali acetate is applied after the application of the metal precursor compounds and/or after the reduction of the metal components of the precursor compounds.

The alkali acetate to be used can be lithium acetate, sodium acetate, potassium acetate, caesium acetate or rubidium acetate, but preferably potassium acetate.

To apply the alkali acetate in the method according to the invention, a solution containing the alkali acetate is preferably produced. Water is preferably used, more preferably deionized water, as solvent for producing the solution. The concentration of the alkali acetate in the solution preferably lies in the range of from 0.5 to 5 mol/L, more preferably 1 to 3 mol/L, even more preferably 1.5 to 2.5 mol/L and most preferably 2 mol/L.

The application of the solution containing the alkali acetate can be carried out in any manner. The application can be carried out by the pore-filling method (incipient wetness) known in the state of the art, but also by other methods, wherein however the application by the pore-filling method before step (a) is preferred according to the invention. Alternatively, the support body can also be coated with alkali acetate by spray coating. The support body can be present static, but is preferably moved. The movement of the support bodies can take place in any conceivable way, for example mechanically with a coating drum, mixing drum or also with the help of a support gas. It is preferred that the movement of the support bodies during the spray coating is carried out with the help of a support gas (or process gas), for example in a fluid bed, a fluidized bed or in an Innojet AirCoater, wherein hot air is preferably blown in, with the result that the solvent is quickly evaporated. The temperature here is preferably 15 to 80°C, more preferably 20 to 40°C and most preferably 30 to 40°C.

If the spray coating of the alkali acetate is carried out before the coating with the metal precursor compounds, the advantage lies in the fact that a torus-shaped alkali-containing structure forms in the volume of the support body, wherein the surface layer of the support body which later bears noble metal remains almost free of alkali metal and thus the noble metals can be taken up in the second application step. If the alkali acetate is applied by spray coating, the spraying rate is preferably chosen during the spraying such that a balance is achieved between the evaporation rate of the solvent and the feed rate of the precursor compounds on the support body. It is particularly preferred that the spraying rate is constant during the spraying-in of the solution containing the alkali acetate and lies in the range of the mass flow of from 5 to 25 g (solution)/min per 100 g of support body to be coated, more preferably in the range of from 10 to 20 g/min per 100 g and most preferably in the range of from 13 to 17 g/min per 100 g. The solution containing alkali acetate is preferably sprayed through a spray nozzle into the apparatus, in which the spray gas fed in, preferably air, is fed in at a pressure in the range of from 1 to 1.8 bar, more preferably 1.0 to 1.6 bar and most preferably 1.1 to 1.3 bar. Process air is preferably used as process gas for circulating the support bodies. The spraying rate and the pressure of the spray gas is preferably chosen for the nozzle used such that when it meets the circulating support bodies the droplet size of the resultant aerosol lies between 1 and 100 pm, preferably between 10 and 40 µm. An IRN10 PEEK-type Rotojet spray nozzle from Innojet is preferably used here.

The alkali metal loading preferably lies in the range of from 2 to 3.5 wt.-%, more preferably 2.2 to 3.0 wt.-% and most preferably 2.5 to 2.7 wt.-%, relative to the total weight of the support body dried after the application.

After the application of the solution containing the alkali acetate, a drying is preferably carried out in the temperature range of from 70 to 120°C, more preferably 80 to 110°C and most preferably 90 to 100°C in air, lean air or inert gas. The duration of the drying of the support bodies loaded with alkali metal preferably lies in the range of from 10 to 100 minutes, more preferably 30 to 60 minutes. The drying of the support body loaded with alkali metal is preferably likewise carried out in the coating device. The drying preferably takes place such that the support bodies are present static in the coating device, i.e. they are not moved, i.e. for example if a fluid bed or fluidized bed device is used the support bodies are preferably not moved in the fluid bed or fluidized bed during the drying.

In an embodiment of the present invention, it is particularly preferred that the support bodies are coated with alkali acetate before step (a) by means of the pore-filling method. The support bodies are then introduced into a coating device. The support bodies are then preferably kept swirling in a fluid bed or a fluidized bed with the help of process air as support gas in order to remove dust from them. The pregilding by spraying in a solution containing an Au precursor compound onto the fluidized support bodies is then preferably started at temperatures of approximately 70°C. When a temperature of approximately 80°C is reached, a switch is made to the spraying-in of a mixed solution or the spraying-in of two solutions containing the Pd precursor compound and Au precursor compound. Immediately afterwards, an aftergilding can take place by spraying in a solution containing an Au precursor compound at the same temperatures. The temperature is then increased to 85 to 100°C and the support bodies are preferably dried static in the coating device. The reduction then takes place according to one of the above-specified steps, but preferably with forming gas while the support bodies are present static.

The shell catalyst obtained by the method according to the invention differs from conventional shell catalysts for the synthesis of VAM in that it has a significantly higher selectivity and activity in the synthesis of VAM. This is to be attributed to the lower metal abrasion during the production using the method according to the invention which leads, not only to a higher metal loading, but also to a lower blockage of the pores due to the dust forming during abrasion. The differences clearly present in respect of the better selectivity and activity of the shell catalyst compared with conventional catalysts cannot be expressed in physical values at the time of the application. The shell catalyst can therefore only be distinguished from conventional catalysts by the manner of its production and the established increased selectivity and activity.

The invention is described in more detail below using two figures and embodiment examples without these being understood as limiting.

Figure:
- Figure 1:: Figure 1 shows the quantity of VAM molecules produced per molecule of oxygen as a function of the CO₂/O₂ ratio when a shell catalyst produced according to the invention and two comparison catalysts are used in the catalytic synthesis of VAM.
- Figure 2:: Figure 2 shows the space-time yield (STY) of VAM as a function of the O₂ conversion when a shell catalyst produced according to the invention and two comparison catalysts are used in the catalytic synthesis of VAM.

### Examples:

### Example 1: Production of a catalyst A:

Firstly, 100 g of the support "KA-Zr-14" (with 14% ZrO₂-doped KA-160 support from Süd-Chemie) was calcined for 4 h at 750°C. Then, the support bodies were presented in a fluid bed in an AirCoater 025-type coater from Innojet using air as process gas for 10 min to remove dust. 20.32 g of a 2 molar KOAc solution was then sprayed onto the swirled support bodies at a spraying rate of 15 g/min/100 g support bodies at 33°C. Then, the support bodies were presented static and dried at 88°C for 35 min. Renewed fluidizing of the support bodies after the drying took place again with the help of air as process gas. Firstly, an aqueous solution containing KAuO₂ (produced by mixing 4.05g of a 3.6% Au solution + 50 ml water) was sprayed onto the swirled support bodies at 70°C at a spraying rate of 15 g solution/min/100 g support bodies. Directly afterwards, two aqueous solutions were sprayed in parallel, wherein one solution contains KAuO₂ (produced by mixing 4.05 g of a 3.6% Au solution with 50 ml water) and the other solution contains Pd(NH₃)₄(OH)₂ (produced by mixing 32.58 g of a 4.7% Pd solution + 50 ml water). Both solutions were sprayed in at a spraying rate of approximately 15 g solution/min/100 g support bodies at a temperature of 70°C. Directly afterwards, an aqueous solution containing KAuO₂ (produced by mixing 4.05 g of a 3.6% Au solution + 50 ml water) was sprayed on at 70°C at a spraying rate of 15 g solution/min/100 g support bodies. Then, the support bodies were presented static in the coater again and dried in this state at 90°C for 35 min. The support bodies were then reduced for 25 min at 70°C with forming gas (98% N₂ and 2% H₂).

According to ICP-AES analysis, the proportion of Pd was 1.4 wt.-% and the proportion of Au was 0.4 wt.-%, in each case relative to the total mass of the support. The noble metal yield for Pd and Au was in each case 97% of the quantity used.

### Comparison example 1: Production of a catalyst B:

Firstly, 100 g of the support "KA-Zr-14" (with 14% ZrO₂-doped KA-160 support from Süd-Chemie) was calcined for 4 h at 750°C. Then, the support bodies were transferred to an AirCoater 025-type coater and presented fluidized with the help of air as process gas. Firstly, an aqueous solution containing KAuO₂ (produced by mixing 4.05 g of a 3.6% Au solution + 50 ml water) was sprayed onto the swirled support bodies at 70°C at a spraying rate of 15 g solution/min/100 g support bodies. Directly afterwards, two aqueous solutions were sprayed in parallel, wherein one solution contains KAuO₂ (produced by mixing 4.05 g of a 3.6% Au solution with 50 ml water) and the other solution contains Pd(NH₃)₄(OH)₂ (produced by mixing 32.58 g of a 4.7% Pd solution + 50 ml water). Both solutions were sprayed in at a spraying rate of approximately 15 g solution/min/100 g support bodies at a temperature of 70°C. Directly afterwards, an aqueous solution containing KAuO₂ (produced by mixing 4.05 g of a 3.6% Au solution + 50 ml water) was sprayed on at 70°C at a spraying rate of 15 g solution/min/100 g support bodies. Then, the support bodies were presented static in the coater again and dried in this state at 90°C for 35 min.

Then, the support bodies were removed from the coater and transferred to a reduction furnace in which they were reduced for 4 h at 150°C with H₂.

Then, the support bodies were impregnated with KOAc by means of the incipient wetness method, by allowing a 2-molar KOAc solution to work on them.

According to ICP-AES analysis, the proportion of Pd was 1.4 wt.-% and the proportion of Au was 0.4 wt.-%, in each case relative to the total mass of the support. The noble metal yield for Pd and Au was in each case 94% of the quantity used.

### Comparison example 2: Production of a catalyst C:

32.58 g Pd(NH₃)₄(OH)₂ solution (4.7%) and 8.10 g KAuO₂ solution (3.6%) were applied as mixed solution to a KA-160 support (obtainable from Süd-Chemie AG) at a spraying rate of 15 g solution per minute per 100 g support bodies at a temperature of 70°C. The support bodies were swirled in an AirCoater 025-type Innojet Air-Coater by means of air as process gas. The obtained support was dried at 90°C for 45 minutes in the static state in the fluidized bed drier. Then, the sample was reduced at 150°C for 4 hours in the gas phase with forming gas (3 vol.-% H₂ in N₂) in a separate reduction reactor. After the reduction, the catalyst was impregnated with a KOAc solution (1.95 g 2 molar KOAc solution and 3.99 g distilled water) according to the incipient wetness method. The sample was then dried at 90°C for 45 minutes in the static state in the fluidized bed drier.

According to ICP-AES analysis, the proportion of Pd was 1.4 wt.-% and the proportion of Au was 0.4 wt.-%, in each case relative to the total mass of the support. The noble metal yield was, for Pd and Au in each case, approx. 92% of the quantity used.

### Comparison example 3: Production of catalysts according to WO 2008/145393:

Catalysts were produced according to the examples of WO 2008/145393. The noble metal yield of these catalysts was, on average, 88% for Pd and 85% for Au.

### Example 2: Test results for catalysts A to C in respect of their activity and selectivity in the synthesis of VAM:

For this, acetic acid, ethylene and oxygen were each passed over the catalysts A and B at a temperature of 140°C/12 hours -> 142°C/12 hours → 144°C/12 h → 146°C/12 hours (these are the respective reaction temperatures that apply according to the sequence during the automated execution of the screening protocol, i.e. measurement is carried out for 12 hours at 140°C, then for 12 hours at 142°C, then for 12 hours at 144°C, and then for 12 hours at 146°C reactor temperature) and a pressure of 6 bar. The concentrations of the components used were: 38% ethylene, 5% O₂, 0.9% CO₂, 9% methane, 12% acetic acid, remainder N₂.

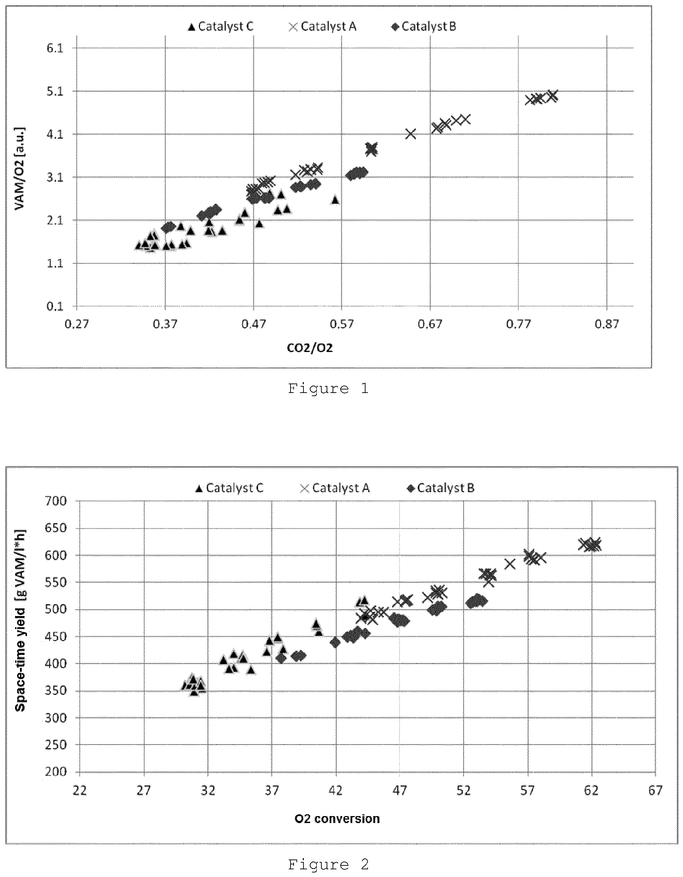

Figures 1 and 2 show the selectivity or the activity of the catalysts A, B and C as a function of the O₂ conversion. The values are also listed in tabular form in the following Tables 1, 2 and 3:

**Table 1:**

| Catalyst A | | | |
|---|---|---|---|
| CO₂/O₂ | VAM/O₂ | O₂ conversion | Space-time yield (g VAM/1h) |
| 0.5 | 2.7 | 44.9 | 480.7 |
| 0.5 | 2.7 | 44.7 | 486.7 |
| 0.5 | 2.7 | 44.0 | 483.1 |
| 0.5 | 2.8 | 44.2 | 492.9 |
| 0.5 | 2.8 | 45.3 | 494.8 |
| 0.5 | 2.8 | 45.7 | 494.6 |
| 0.5 | 2.8 | 44.7 | 497.9 |
| 0.5 | 3.3 | 49.8 | 530.1 |
| 0.5 | 3.3 | 50.1 | 535.7 |
| 0.5 | 3.3 | 50.3 | 531.0 |
| 0.5 | 3.3 | 49.8 | 534.0 |
| 0.5 | 3.2 | 50.0 | 527.7 |
| 0.5 | 3.1 | 49.2 | 521.8 |
| 0.6 | 3.8 | 53.7 | 566.9 |
| 0.6 | 3.8 | 54.2 | 562.7 |
| 0.6 | 3.8 | 54.1 | 565.9 |
| 0.6 | 3.8 | 54.0 | 563.7 |
| 0.6 | 3.7 | 53.9 | 550.4 |
| 0.6 | 3.8 | 53.6 | 566.7 |
| 0.6 | 4.1 | 55.6 | 583.9 |
| 0.7 | 4.5 | 58.0 | 595.1 |
| 0.7 | 4.4 | 57.1 | 601.4 |
| 0.7 | 4.4 | 57.1 | 598.5 |
| 0.7 | 4.3 | 57.5 | 592.3 |
| 0.7 | 4.3 | 57.5 | 591.5 |
| 0.7 | 4.2 | 57.3 | 593.2 |
| 0.8 | 5.0 | 62.3 | 623.1 |
| 0.8 | 5.0 | 62.1 | 617.4 |
| 0.8 | 5.0 | 62.4 | 617.8 |
| 0.8 | 4.9 | 61.9 | 617.1 |
| 0.8 | 4.9 | 61.8 | 615.9 |

**Table 2:**

| Catalyst B | | | |
|---|---|---|---|
| CO/O2 | VAM/O2 | O2 conversion | Space-time yield (g VAM/1h) |
| 0.37 | 1.91 | 37.68 | 410.4 |
| 0.37 | 1.94 | 38.9 | 413.84 |
| 0.38 | 1.96 | 39.28 | 414.82 |
| 0.42 | 2.26 | 42.89 | 449.37 |
| 0.42 | 2.27 | 43.41 | 448.08 |
| 0.42 | 2.28 | 43.15 | 451.97 |
| 0.42 | 2.29 | 43.42 | 451.07 |
| 0.43 | 2.35 | 43.68 | 459.91 |
| 0.43 | 2.35 | 44.28 | 455.77 |
| 0.47 | 2.58 | 46.83 | 477.55 |
| 0.47 | 2.61 | 47.09 | 480.28 |
| 0.47 | 2.61 | 46.56 | 484.24 |
| 0.47 | 2.62 | 47.22 | 480.06 |
| 0.48 | 2.62 | 47.36 | 479.17 |
| 0.48 | 2.62 | 46.94 | 481.09 |
| 0.48 | 2.62 | 46.82 | 480.75 |
| 0.53 | 2.93 | 49.97 | 505.31 |
| 0.54 | 2.95 | 50.27 | 504.67 |
| 0.54 | 2.95 | | 502.98 |
| 0.52 | 2.99 | | 498.56 |
| 0.52 | 2.88 | | 498.85 |
| 0.52 | 2.87 | 49.55 | 498.99 |
| 0.52 | 2.88 | 49.89 | 497.08 |
| 0.59 | 3.21 | 53.17 | 517.84 |
| 0.59 | 3.22 | 53.45 | 515.59 |
| 0.59 | 3.19 | 53.03 | 516.06 |
| 0.58 | 3.19 | 52.94 | 515.34 |
| 0.59 | 3.22 | 53.04 | 519.72 |
| 0.58 | 3.17 | 52.73 | 513.56 |
| 0.58 | 3.14 | 52.55 | 511.64 |
| 0.41 | 2.2 | 41.95 | 439.28 |

**Table 3:**

| Catalyst C | | | |
|---|---|---|---|
| CO/O2 | VAM/O2 | O2 conversion | Space-time yield [g VAM/1h] |
| 0.31 | 1.21 | 27.35 | 303.85 |
| 0.35 | 1.25 | 28.47 | 309.68 |
| 0.37 | 1.38 | 29.04 | 339.25 |
| 0.34 | 1.44 | 28.80 | 354.44 |
| 0.34 | 1.35 | 28.85 | 331.39 |
| 0.36 | 1.44 | 29.89 | 347.01 |
| 0.34 | 1.42 | 29.84 | 343.49 |
| 0.39 | 1.43 | 30.57 | 342.34 |
| 0.34 | 1.46 | 30.56 | 349.29 |
| 0.36 | 1.48 | 30.75 | 352.59 |
| 0.38 | 1.51 | 31.61 | 354.71 |
| 0.36 | 1.53 | 31.58 | 360.97 |
| 0.41 | 1.83 | 34.85 | 411.48 |
| 0.38 | 1.71 | 34.04 | 386.60 |
| 0.43 | 1.86 | 35.19 | 412.62 |
| 0.42 | 2.03 | 37.70 | 433.66 |
| 0.44 | 2.01 | 38.35 | 424.94 |
| 0.51 | 2.36 | 41.48 | 468.41 |
| 0.46 | 2.36 | 41.41 | 469.33 |
| 0.52 | 2.28 | 41.80 | 450.27 |
| 0.49 | 2.39 | 41.55 | 474.80 |
| 0.51 | 2.64 | 45.47 | 486.80 |
| 0.38 | 1.94 | 34.97 | 424.49 |
| 0.39 | 1.87 | 34.89 | 412.60 |
| 0.39 | 1.81 | 35.26 | 398.06 |

As can be seen from the comparison of the values from Tables 1 to 3 and Figures 1 and 2, the catalyst A produced according to the invention has a substantially higher selectivity and activity (O₂ conversion rate) than the comparison catalysts B and C.

## Claims

1. Method for producing a shell catalyst, comprising the following steps:
(a) introducing a support body comprising an Si-Al mixed oxide into a coating device;
(b) applying a Pd precursor compound and an Au precursor compound, in each case in dissolved form, to the support body by spray coating in the coating device;
(c) drying the support body coated with the precursor compounds in the coating device;
(d) reducing the metal components of the precursor compounds to the elemental metals in the coating device; and
(e) removing the support body from the coating device,
wherein the support body remains in the coating device between steps (a) and (e) and wherein an alkali acetate is applied to the support body before the application of the Pd precursor compound and Au precursor compound in step (b).

2. Method according to claim 1, wherein the Pd precursor compound is a hydroxo complex.

3. Method according to claim 1 or 2, wherein the Au precursor compound is an aurate.

4. Method according to one of claims 1 to 3, wherein step (b) is carried out by simultaneous application of the Pd precursor compound and the Au precursor compound.

5. Method according to one of claims 1 to 4, wherein the application of the Pd precursor compound and of the Au precursor compound in step (b) takes place either by spray coating of a mixed solution containing both precursor compounds or by spray coating of two solutions each containing one of the precursor compounds.

6. Method according to one of claims 1 to 5, wherein an Au precursor compound is additionally applied to the support body between steps (b) and (c).

7. Method according to claim 6, wherein the application of the Au precursor compound takes place by spray coating of a solution containing the precursor compound.

8. Method according to one of claims 1 to 7, wherein the support bodies are swirled in a glide layer of process gas during the spray coating.

9. Method according to one of claims 1 to 8, wherein the device is a fluid bed device or a fluidized bed unit.

10. Method according to one of claims 1 to 9, wherein, after step (a) and before the spray coating with the metal precursor compounds, a step of removing dust from the support body is carried out by swirling the support body in a glide layer of process gas.

11. Method according to one of claims 1 to 10, wherein the support body is present static in the coating device during the step (c) of drying.

12. Method according to one of claims 1 to 11, wherein the reduction of the metal components is carried out in a non-oxidizing atmosphere.

13. Method according to claim 12, wherein the non-oxidizing atmosphere contains hydrogen or ethylene as reducing agent.

14. Method according to one of claims 1 to 13, wherein the support body is present static in the coating device during the step (d) of reduction.

## Patentansprüche

1. Verfahren zur Herstellung eines Schalenkatalysators umfassend die folgenden Schritte:
(a) Einbringen eines Trägerkörpers umfassend ein Si-Al-Mischoxid in eine Beschichtungsvorrichtung;
(b) Aufbringen einer Pd-Vorläuferverbindung und einer Au-Vorläuferverbindung jeweils in gelöster Form auf den Trägerkörper durch Sprühbeschichten in der Beschichtungsvorrichtung;
(c) Trocknens des mit den Vorläuferverbindungen beschichteten Trägerkörpers in der Beschichtungsvorrichtung;
(d) Reduktion der Metallkomponenten der Vorläuferverbindungen zu den elementaren Metallen in der Beschichtungsvorrichtung; und
(e) Ausbringen des Trägerkörpers aus der Beschichtungsvorrichtung,
wobei der Trägerkörper zwischen den Schritten (a) und (e) in der Beschichtungsvorrichtung verbleibt und wobei vor dem Aufbringen der Pd-Vorläuferverbindung und der Au-Vorläuferverbindung in Schritt (b) ein Alkaliacetat auf den Trägerkörper aufgebracht wird.

2. Verfahren nach Anspruch 1, worin die Pd-Vorläuferverbindung ein Hydroxo-Komplex ist.

3. Verfahren nach Anspruch 1 oder 2, worin die Au-Vorläuferverbindung ein Aurat ist.

4. Verfahren nach einem der Ansprüche 1 bis 3, worin der Schritt (b) durch zeitgleiches Aufbringen der Pd-Vorläuferverbindung und der Au-Vorläuferverbindung durchgeführt wird.

5. Verfahren nach einem der Ansprüche 1 bis 4, worin das Aufbringen der Pd-Vorläuferverbindung und der Au-Vorläuferverbindung in Schritt (b) entweder durch Sprühbeschichten einer Mischlösung enthaltend beide Vorläuferverbindungen oder durch Sprühbeschichten zweier Lösungen jeweils enthaltend eine der Vorläuferverbindungen stattfindet.

6. Verfahren nach einem der Ansprüche 1 bis 5, worin zwischen den Schritten (b) und (c) zusätzlich eine Au-Vorläuferverbindung auf den Trägerkörper aufgebracht wird.

7. Verfahren nach Anspruch 6, worin das Aufbringen der Au-Vorläuferverbindung durch Sprühbeschichten einer Lösung enthaltend die Vorläuferverbindung stattfindet.

8. Verfahren nach einem der Ansprüche 1 bis 7, worin die Trägerkörper während dem Sprühbeschichten in einer Gleitschicht aus Prozessgas verwirbelt werden.

9. Verfahren nach einem der Ansprüche 1 bis 8, worin die Vorrichtung eine Fließbettvorrichtung oder eine Wirbelschichtanlage ist.

10. Verfahren nach einem der Ansprüche 1 bis 9, worin nach dem Schritt (a) und vor dem Sprühbeschichten mit den Metall-Vorläuferverbindungen ein Schritt des Entstaubens des Trägerkörpers durchgeführt wird, indem der Trägerkörper in einer Gleitschicht aus Prozessgas verwirbelt wird.

11. Verfahren nach einem der Ansprüche 1 bis 10, worin der Trägerkörper während dem Schritt (c) des Trocknens statisch in der Beschichtungsvorrichtung vorliegt.

12. Verfahren nach einem der Ansprüche 1 bis 11, worin die Reduktion der Metallkomponenten in einer nichtoxidierenden Atmosphäre durchgeführt wird.

13. Verfahren nach Anspruch 12, worin die nichtoxidierende Atmosphäre als Reduktionsmittel Wasserstoff oder Ethylen enthält.

14. Verfahren nach einem der Ansprüche 1 bis 13, worin der Trägerkörper während dem Schritt (d) der Reduktion statisch in der Beschichtungsvorrichtung vorliegt.

## Revendications

1. Procédé de fabrication d'un catalyseur à coque, comprenant les étapes suivantes :
(a) l'introduction d'un corps de support comprenant un oxyde mixte de Si-Al dans un dispositif de revêtement ;
(b) l'application d'un composé de précurseur de Pd et d'un composé de précurseur d'Au, dans chaque cas sous forme dissoute, sur le corps de support par revêtement par pulvérisation dans le dispositif de revêtement ;
(c) le séchage du corps de support revêtu avec les composés de précurseurs dans le dispositif de revêtement ;
(d) la réduction des composants métalliques des composés de précurseurs en métaux élémentaires dans le dispositif de revêtement ; et
(e) le retrait du corps de support du dispositif de revêtement,
dans lequel le corps de support reste dans le dispositif de revêtement entre les étapes (a) et (e) et dans lequel un acétate alcalin est appliqué sur le corps de support avant l'application du composé de précurseur de Pd et du composé de précurseur d'Au à l'étape (b).

2. Procédé selon la revendication 1, dans lequel le composé de précurseur de Pd est un complexe hydroxo.

3. Procédé selon la revendication 1 ou 2, dans lequel le composé de précurseur d'Au est un aurate.

4. Procédé selon l'une des revendications 1 à 3, dans lequel l'étape (b) est réalisée par l'application simultanée du composé de précurseur de Pd et du composé de précurseur d'Au.

5. Procédé selon l'une des revendications 1 à 4, dans lequel l'application du composé de précurseur de Pd et du composé de précurseur d'Au à l'étape (b) a lieu soit par revêtement par pulvérisation d'une solution mixte contenant les deux composés de précurseurs, soit par revêtement par pulvérisation de deux solutions contenant chacune un des composés de précurseurs.

6. Procédé selon l'une des revendications 1 à 5, dans lequel un composé de précurseur d'Au est en outre appliqué sur le corps de support entre les étapes (b) et (c).

7. Procédé selon la revendication 6, dans lequel l'application du composé de précurseur d'Au a lieu par revêtement par pulvérisation d'une solution contenant le composé de précurseur.

8. Procédé selon l'une des revendications 1 à 7, dans lequel les corps de support sont mis à tournoyer dans une couche de glissement d'un gaz de traitement pendant le revêtement par pulvérisation.

9. Procédé selon l'une des revendications 1 à 8, dans lequel le dispositif est un dispositif à lit fluidisé ou une unité à lit fluidisé.

10. Procédé selon l'une des revendications 1 à 9, dans lequel, après l'étape (a) et avant le revêtement par pulvérisation avec les composés de précurseurs métalliques, une étape d'élimination de la poussière présente sur le corps de support est réalisée en faisant tournoyer le corps de support dans une couche de glissement d'un gaz de traitement.

11. Procédé selon l'une des revendications 1 à 10, dans lequel le corps de support est présent et statique dans le dispositif de revêtement pendant l'étape (c) de séchage.

12. Procédé selon l'une des revendications 1 à 11, dans lequel la réduction des composants métalliques est réalisée dans une atmosphère non oxydante.

13. Procédé selon la revendication 12, dans lequel l'atmosphère non oxydante contient de l'hydrogène ou de l'éthylène en tant qu'agent réducteur.

14. Procédé selon l'une des revendications 1 à 13, dans lequel le corps de support est présent et statique dans le dispositif de revêtement pendant l'étape (d) de réduction.
